(19) **Europäisches Patentamt**
**European Patent Office**
**Office européen des brevets**

(11) **EP 4 336 510 A1**

(12) **EUROPEAN PATENT APPLICATION**

(43) Date of publication:
**13.03.2024 Bulletin 2024/11**

(51) International Patent Classification (IPC):
**G16H 20/17** (2018.01)     **G16H 40/63** (2018.01)

(21) Application number: **23193193.2**

(52) Cooperative Patent Classification (CPC):
**G16H 20/17; G16H 40/63;** G16H 20/10

(22) Date of filing: **24.08.2023**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.08.2022 US 202263373388 P**

(71) Applicant: **Insulet Corporation**
**Acton MA 01720 (US)**

(72) Inventors:
• **LEE, Joon Bok**
  **Acton (US)**
• **D'ARCO, John**
  **Wilmington (US)**
• **O'CONNOR, Jason**
  **Acton (US)**

(74) Representative: **Peterreins Schley**
**Patent- und Rechtsanwälte PartG mbB**
**Hermann-Sack-Straße 3**
**80331 München (DE)**

(54) **SYSTEM AND METHOD FOR ADJUSTING INSULIN DELIVERY TO ACCOUNT FOR INSULIN RESISTANCE**

(57)     Disclosed herein is a method, implemented in an automatic drug delivery system, for determining insulin resistance of a user and adjusting parameters of the medication delivery algorithm of the automatic drug delivery system based on the determined insulin resistance. A self-guided test is administered similar to the Kraft test which determines if the quantity of insulin delivered during a post-prandial period after ingestion of a quantity of fast-acting carbohydrates is sufficient to lower the user's blood glucose level to a baseline level.

FIG. 1

**Description**

**BACKGROUND**

Many conventional automatic drug delivery (ADD) systems are well known, including, for example, wearable drug delivery devices. The drug delivery device can be designed to deliver any type of liquid drug to a user. In specific embodiments, the drug delivery device can be, for example, an OmniPod® drug delivery device manufactured by Insulet Corporation of Acton, Massachusetts. The drug delivery device can be a drug delivery device such as those described in U.S. Pat. No. 7,303,549, U.S. Pat. No. 7,137,964, or U.S. Pat. No. 6,740,059, each of which is incorporated herein by reference in its entirety.

Many users of ADD systems experience some degree of insulin resistance. Insulin resistance, also known as impaired insulin sensitivity, occurs when cells in the muscles, fat, and liver do not respond well to insulin and cannot use glucose from the blood for energy. To compensate, the pancreas makes more insulin, a condition known as hyperinsulinemia. Over time, the pancreas may not be able to make enough insulin to overcome the weak response of the cells to insulin, and the user's blood sugar levels rise.

Insulin resistance and sensitivity plays a major part in the glucose and insulin dynamics of people with diabetes, particularly for type 2 patients. Several clinical tests may be utilized to directly measure these factors, and the outcomes of these tests can be utilized to better inform the use of ADD systems for type 2 diabetic users.

There are several tests that can be used to determine a person's insulin resistance. One such test, known as the Kraft test, measures the body's response to a "glucose challenge" (i.e., ingestion of a quantity of a sugary solution) and how long the user's body takes to return to a baseline blood glucose level after initiating the glucose challenge. The test matches the body's response with known patterns predictive of varying degrees of insulin resistance. An alternative set of patterns (e.g., Hayashi patterns) are sometimes used to assess the risk for some users.

Therefore, it would be useful to (1) adjust default parameters of the ADD system controlling the quantity of insulin delivery for new users based on administration of the Kraft test (and/or a Hayashi test, but generally referred to hereafter as a Kraft test) by a health care professional; and (2) have the ADD system guide the user to perform a self-guided insulin resistance test; and (3) adjust parameters of the ADD system controlling the quantity of insulin delivery based on the results of the self-guided test.

**SUMMARY**

This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

In a typical ADD system, such as the one described herein, parameters of the ADD system controlling the quantity of insulin delivered to the user can be adjusted for new users of the ADD system based on the results of a Kraft test performed on the user by a health care professional. Therefore, in a first aspect of the invention, disclosed herein is a method for adjusting parameters of the ADD system based on the results of a Kraft test. The outcome of such glucose tolerance tests with insulin assay can be utilized to establish a better initial parametrization of the ADD system and improve glucose control outcomes for these users.

In a second aspect of the invention, the user can be assisted to perform a self-guided insulin resistance test similar to the Kraft test. The result of the self-guided test can be used to automatically adjust various parameters of the ADD system controlling the quantity of insulin delivered to the user.

**BRIEF DESCRIPTION OF THE DRAWINGS**

In the drawings, like reference characters generally refer to the same parts throughout the different views. In the following description, various embodiments of the present invention are described with reference to the following drawings, in which:

FIG. 1 illustrates a functional block diagram of an exemplary system suitable for use with the methods disclosed herein.
FIGS. 2(a-b) show patterns used to categorize outcomes of the Kraft test.
FIG. 3 is a flow chart showing the process for conducting the self-guided insulin resistance test described herein.

**DETAILED DESCRIPTION**

Various embodiments of the present invention include systems and methods for delivering a medication to a user using a drug delivery device, either autonomously, or in accordance with a wireless signal received from an electronic device. In various embodiments, the electronic device may be a user device comprising a smartphone, a smart watch, a smart necklace, a module attached to the drug delivery device, or any other type or sort of electronic device that may be carried by the user or worn on the body of the user and that can execute an algorithm that computes the times and dosages of delivery of the medication. Alternatively, the drug delivery device operates an algorithm stored on the drug delivery device itself, without reliance on a remote electronic device for delivering medicament to the user.

For example, the user device or drug delivery device may execute an "artificial-pancreas" (AP) algorithm that computes the times and dosages of delivery of insulin. The user device and/or drug delivery device may also be in communication with a sensor, such as a glucose sensor or a continuous glucose monitor (CGM), that collects data on a physical attribute or condition of the user, such as a glucose level. The sensor may be disposed in or on the body of the user and may be part of the drug delivery device or may be a separate device.

Alternatively, the drug delivery device may be in communication with the sensor in lieu of or in addition to the communication between the sensor and the user device. The communication may be direct (if, e.g., the sensor is integrated with or otherwise a part of the drug delivery device) or remote/wireless (if, e.g., the sensor is disposed in a different housing than the drug delivery device). In these embodiments, the drug delivery device contains computing hardware (e.g., a processor, memory, firmware, etc.) that executes some or all of the algorithm that computes the times and dosages of delivery of the medication.

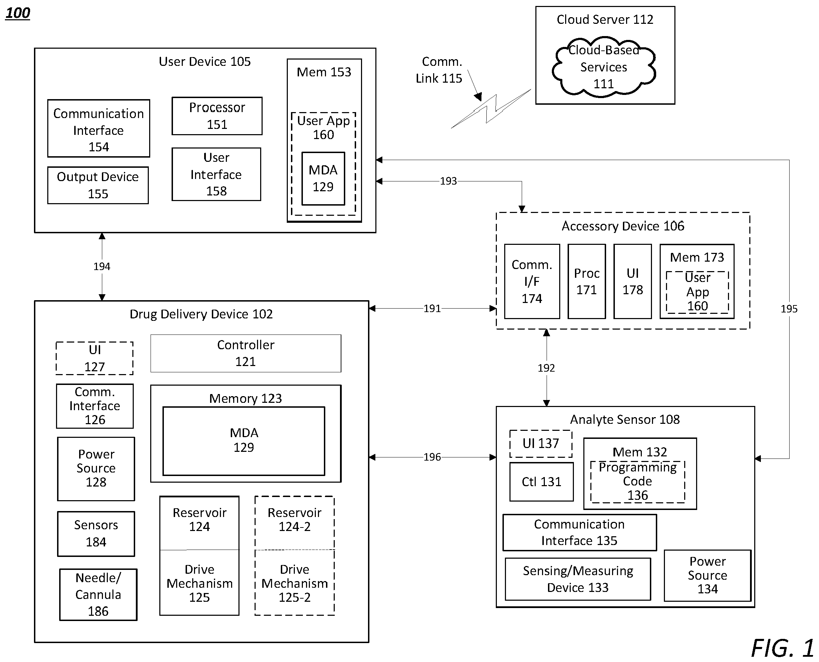

**FIG. 1** illustrates a functional block diagram of an exemplary drug delivery system 100 suitable for implementing the systems and methods described herein. The drug delivery system 100 may implement (and/or provide functionality for) a medication delivery algorithm, such as an artificial pancreas (AP) application, to govern or control the automated delivery of a drug or medication, such as insulin, to a user (e.g., to maintain euglycemia - a normal level of glucose in the blood). The drug delivery system 100 may be an automated drug delivery system that may include a drug delivery device 102 (which may be wearable), an analyte sensor 108 (which may also be wearable), and a user device 105.

Drug delivery system 100, in an optional example, may also include an accessory device 106, such as a smartwatch, a personal assistant device, a smart insulin pen, or the like, which may communicate with the other components of system 100 via either a wired or wireless communication links 191-193.

*User Device*

The user device 105 may be a computing device such as a smartphone, a smartwatch, a tablet, a personal diabetes management (PDM) device, a dedicated diabetes therapy management device, or the like. In an example, user device 105 may include a processor 151, device memory 153, a user interface 158, and a communication interface 154. The user device 105 may also contain analog and/or digital circuitry that may be implemented as a processor 151 for executing processes based on programming code stored in device memory 153, such as user application 160 incorporating medication delivery algorithm (MDA) 129 to manage a user's blood glucose levels and for controlling the delivery of the drug, medication, or therapeutic agent to the user, as well for providing other functions, such as calculating carbohydrate-compensation dosage, a correction bolus dosage and the like as discussed below. The user device 105 may be used to activate, deactivate, trigger a needle/canula insertion, program, adjust settings, and/or control operation of drug delivery device 102 and/or the analyte sensor 103 as well as the optional smart accessory device 106.

The processor 151 may also be configured to execute programming code stored in device memory 153, such as the user app 160. The user app 160 may be a computer application that is operable to deliver a drug based on information received from the analyte sensor 103, the cloud-based services 111 and/or the user device 105 or optional accessory device 106. The memory 153 may also store programming code to, for example, operate the user interface 158 (e.g., a touchscreen device, a camera or the like), the communication interface 154 and the like. The processor 151, when executing user app 160, may be configured to implement indications and notifications related to meal ingestion, blood glucose measurements, and the like. The user interface 158 may be under the control of the processor 151 and be configured to present a graphical user interface that enables the input of a meal announcement, adjust setting selections and the like as described herein.

In a specific example, when the user app 160 includes MDA 129, the processor 151 is also configured to execute a diabetes treatment plan (which may be stored in a memory) that is managed by user app 160. In addition to the functions mentioned above, when user app 160 is an AP application, it may further provide functionality to determine a carbohydrate-compensation dosage, a correction bolus dosage and determine a real-time basal dosage according to a diabetes treatment plan. In addition, as MDA 129, user app 160 provides functionality to output signals to the drug delivery device 102 via communications interface 154 to deliver the determined bolus and/or basal dosages.

The communication interface 154 may include one or more transceivers that operate according to one or more radio-frequency protocols. In one embodiment, the transceivers may comprise a cellular transceiver and a Bluetooth® transceiver. The communication interface 154 may be configured to receive and transmit signals containing information usable by user app 160.

User device 105 may be further provided with one or more output devices 155 which may be, for example, a speaker or a vibration transducer, to provide various signals to the user.

*Drug Delivery Device*

In various exemplary embodiments, drug delivery device 102 may include a reservoir 124 and drive mechanism 125, which are controllable by controller 121, executing a medication delivery algorithm (MDA) 129 stored in memory 123, which may perform some or all of the functions of the AP application described above, such that user device 105 may be unnecessary for drug delivery device 102 to carry out drug delivery and control. MDA 129 excluding on drug delivery device 102 may be identical to MDA 129 executing on user device 105, but without the user interface screens provided by user app 160. Alternatively, the functionality of MDA 129 may be split between user device 105 and drug delivery device 102.

Controller 121 may act to control reservoir 124 and drive mechanism 125 based on signals received from user app 160 executing on a user device 105 and communicated to drug delivery device 102 via communication link 194. Drive mechanism 125 may operate to longitudinally translate a plunger through the reservoir, so as to force the liquid drug through an outlet fluid port to needle / cannula 186. Alternatively, other types of drive mechanisms may be used.

In an alternate embodiment, drug delivery device 102 may also include an optional second reservoir 124-2 and second drive mechanism 125-2 which enables the independent delivery of two different liquid drugs. As an example, reservoir 124 may be filled with insulin, while reservoir 124-2 may be filled with glucagon, or Pramlintide, or GLP-1. In some embodiments, each of reservoirs 124, 124-2 may be configured with a separate drive mechanism 125, 125-2, respectively, which may be separately controllable by controller 121 under the direction of MDA 129. Both reservoirs 124, 124-2 may be connected to a common needle / cannula 186.

Drug delivery device 102 may be optionally configured with a user interface 127 providing a means for receiving input from the user and a means for outputting information to the user. User interface 127 may include, for example, light-emitting diodes, buttons on a housing of drug delivery device 102, a sound transducer, a micro-display, a microphone, an accelerometer for detecting motions of the device or user gestures (e.g., tapping on a housing of the device) or any other type of interface device that is configured to allow a user to enter information and/or allow drug delivery device 102 to output information for presentation to the user (e.g., alarm signals or the like).

Drug delivery device 102 includes a patient interface 186 for interfacing with the user to deliver the liquid drug. Patient interface may be, for example, a needle or cannula for delivering the drug into the body of the user (which may be done subcutaneously, intraperitoneally, or intravenously). Drug delivery device 102 may further include a mechanism for inserting the needle / cannula 186 into the body of the user, which may be integral with or attachable to drug delivery device 102. The insertion mechanism may comprise, in one embodiment, an actuator that inserts the needle / cannula 186 under the skin of the user and thereafter retracts the needle, leaving the cannula in place. The actuator may be triggered by user device 105 or may be a manual firing mechanism comprising springs or other energy storing mechanism, that causes the needle / cannula 186 to penetrate the skin of the user.

In one embodiment, drug delivery device 102 includes a communication interface 126, which may be a transceiver that operates according to one or more radio-frequency protocols, such as Bluetooth®, Wi-Fi, near-field communication, cellular, or the like. The controller 121 may, for example, communicate with user device 105 and an analyte sensor 108 via the communication interface 126.

In some embodiments, drug delivery device 102 may be provided with one or more sensors 184. The sensors 184 may include one or more of a pressure sensor, a power sensor, or the like that are communicatively coupled to the controller 121 and provide various signals. For example, a pressure sensor may be configured to provide an indication of the fluid pressure detected in a fluid pathway between the patient interface 186 and reservoir 124. The pressure sensor may be coupled to or integral with the actuator for inserting the patient interface 186 into the user. In an example, the controller 121 may be operable to determine a rate of drug infusion based on the indication of the fluid pressure. The rate of drug infusion may be compared to an infusion rate threshold, and the comparison result may be usable in determining an amount of insulin onboard (IOB) or a total daily insulin (TDI) amount. In one embodiment, analyte sensor 108 may be integral with drug delivery device 102.

Drug delivery device 102 further includes a power source 128, such as a battery, a piezoelectric device, an energy harvesting device, or the like, for supplying electrical power to controller 121, memory 123, drive mechanisms 125 and/or other components of drug delivery device 102.

Drug delivery device 102 may be configured to perform and execute processes required to deliver doses of the medication to the user without input from the user device 105 or the optional accessory device 106. As explained in more detail, MDA 129 may be operable, for example, to determine an amount of insulin to be delivered, IOB, insulin remaining, and the like and to cause controller 121 to activate drive mechanism 125 to deliver the medication from reservoir 124. MDA 129 may take as input data received from the analyte sensor 108 or from user app 160.

The reservoirs 124, 124-2 may be configured to store drugs, medications or therapeutic agents suitable for automated delivery, such as insulin, Pramlintide, GLP-1, co-formulations of insulin and GLP-1, glucagon, morphine, blood pressure medicines, arthritis drugs, chemotherapy drugs, fertility drugs, hormonal drugs, or the like.

Drug delivery device 102 may be a wearable device and may be attached to the body of a user, such as a patient or diabetic, at an attachment location and may deliver any therapeutic agent, including any drug or medicine, such as insulin or the like, to a user at or around the attachment location. A surface of drug delivery device 102 may include an adhesive to facilitate attachment to the skin of a user.

When configured to communicate with an external device, such as the user device 105 or the analyte sensor 108, drug delivery device 102 may receive signals over the wired or wireless link 194 from the user device 105 or from the analyte sensor 108. The controller 121 of drug delivery device 102 may receive and process the signals from the respective external devices as well as implementing delivery of a drug to the user according to a diabetes treatment plan or other drug delivery regimen.

*Accessory Device*

Optional accessory device 106 may be, a wearable smart device, for example, a smart watch (e.g., an Apple Watch®), smart eyeglasses, smart jewelry, a global positioning system-enabled wearable, a wearable fitness device, smart clothing, or the like. Accessory device 106 may alternatively be a smart insulin pen that works with drug delivery device 102 in managing blood glucose and treating diabetes of a user. Similar to user device 105, the accessory device 106 may also be configured to perform various functions including controlling or communicating with drug delivery device 102. For example, the accessory device 106 may include a communication interface 174, a processor 171, a user interface 178 and a memory 173. The user interface 178 may be a graphical user interface presented on a touchscreen display of the smart accessory device 107. The memory 173 may store programming code to operate different functions of the smart accessory device 107 as well as an instance of the user app 160, or a pared-down version of user app 160 with reduced functionality. In some instances, accessory device 107 may also include sensors of various types.

*Analyte Sensor*

The analyte sensor 108 may include a controller 131, a memory 132, a sensing/measuring device 133, an optional user interface 137, a power source/energy harvesting circuitry 134, and a communication interface 135. The analyte sensor 108 may be communicatively coupled to the processor 151 of the management device 105 or controller 121 of drug delivery device 102. The memory 132 may be configured to store information and programming code 136.

The analyte sensor 108 may be configured to detect one or multiple different analytes, such as glucose, lactate, ketones, uric acid, sodium, potassium, alcohol levels or the like, and output results of the detections, such as measurement values or the like. The analyte sensor 108 may, in an exemplary embodiment, be configured as a continuous glucose monitor (CGM) to measure a blood glucose values at a predetermined time interval, such as every 5 minutes, every 1 minute, or the like. The communication interface 135 of analyte sensor 108 may have circuitry that operates as a transceiver for communicating the measured blood glucose values to the user device 105 over a wireless link 195 or with drug delivery device 102 over the wireless communication link 108. While referred to herein as an analyte sensor 108, the sensing/measuring device 133 of the analyte sensor 108 may include one or more additional sensing elements, such as a glucose measurement element, a heart rate monitor, a pressure sensor, or the like. The controller 131 may include discrete, specialized logic and/or components, an application-specific integrated circuit, a microcontroller or processor that executes software instructions, firmware, programming instructions stored in memory (such as memory 132), or any combination thereof.

Similar to the controller 121 of drug delivery device 102, the controller 131 of the analyte sensor 108 may be operable to perform many functions. For example, the controller 131 may be configured by programming code 136 to manage the collection and analysis of data detected by the sensing and measuring device 133.

Although the analyte sensor 108 is depicted in **FIG. 1** as separate from drug delivery device 102, in various embodiments, the analyte sensor 108 and drug delivery device 102 may be incorporated into the same unit. That is, in various examples, the analyte sensor 108 may be a part of and integral with drug delivery device 102 and contained within the same housing as drug delivery device 102 or an attachable housing thereto. In such an example configuration, the controller 121 may be able to implement the functions required for the proper delivery of the medication alone without any external inputs from user device 105, the cloud-based services 111, another sensor (not shown), the optional accessory device 106, or the like.

*Cloud-Based Services*

Drug delivery system 100 may communicate with or receive services from a cloud server 122 providing cloud-based services 111. Services provided by cloud server 112 may include data storage that stores personal or anonymized data, such as blood glucose measurement values, historical IOB or TDI, prior carbohydrate-compensation dosage, and other forms of data. In addition, the cloud-based services 111 may process anonymized data from multiple users to provide generalized information related to TDI, insulin sensitivity, IOB and the like. The communication link 115 that couples the cloud server 112 to other components of system 100, for example, devices 102, 105, 106, 108 of system 100 may be a cellular link, a Wi-Fi link, a Bluetooth® link, or a combination thereof.

*Communication Links*

The wireless communication links 115 and 191-196 may be any type of wireless link operating using known wireless communication standards or proprietary standards. As an example, the wireless communication links 191-196 may provide communication links based on Bluetooth®, Zigbee®, Wi-Fi, a near-field communication standard, a cellular standard, or any other wireless protocol via the respective communication interfaces 126, 135, 154 and 174.

*Operational Example*

In an operational example, user application 160 implements a graphical user interface that is the primary interface with the user and is used to activate drug delivery device 102, trigger a needle/cannula insertion, start and stop drug delivery device 102, program basal and bolus calculator settings for manual mode as well as program settings specific for automated mode (hybrid closed-loop or closed-loop).

User app 160, provides a graphical user interface 158 that allows for the use of large text, graphics, and on-screen instructions to prompt the user through the set-up processes and the use of system 100. It may also be used to program the user's custom basal insulin delivery profile, accept a recommended basal insulin delivery profile, check the status of drug delivery device 102, initiate bolus doses of insulin, make changes to a patient's insulin delivery profile, handle system alerts and alarms, or allow the user to switch between automated mode and manual mode.

User app 160 may be configured to operate in a manual mode in which user app 160 will deliver insulin at programmed basal rates and user-defined bolus amounts with the option to set temporary basal profiles. The controller 121 will also have the ability to function as a sensor-augmented pump in manual mode, using sensor glucose data provided by the analyte sensor 108 to populate the bolus calculator.

User app 160 may be configured to operate in an automated mode in which user app 160 supports the use of one or multiple target blood glucose values that may be adjusted manually or automatically by the system. For example, in one embodiment, target blood glucose values can range from 110-150 mg/dL, in 10 mg/dL increments, in 5 mg/dL increments, or other increments, but preferably 10 mg/dL increments. The experience for the user will reflect current setup flows whereby the healthcare provider assists the user to program basal rates, glucose targets and bolus calculator settings. These in turn will inform the user app 160 for insulin dosing parameters. The insulin dosing parameters will be adapted over time based on the total daily insulin (TDI) delivered during each use of drug delivery device 102. A temporary hypoglycemia protection mode or an activity mode may be implemented by the user for various time durations in automated mode. With hypoglycemia protection mode or an activity mode, the algorithm reduces insulin delivery and is intended for use over temporary durations when insulin sensitivity is expected to be higher, such as during exercise or fasting.

The user app 160 (or MDA 129) may provide periodic insulin micro-boluses based upon past glucose measurements and/or a predicted glucose over a prediction horizon (e.g., 60 minutes). Optimal post-prandial control may require the user to give meal boluses in the same manner as current pump therapy, but normal operation of the user app 160 will compensate for missed meal boluses and mitigate prolonged hyperglycemia. The user app 160 uses a control-to-target strategy that attempts to achieve and maintain a set target glucose value, thereby reducing the duration of prolonged hyperglycemia and hypoglycemia.

In some embodiments, user device 105 and the analyte sensor 108 may not communicate directly with one another. Instead, data (e.g., blood glucose readings) from analyte sensor may be communicated to drug delivery device 102 via link 196 and then relayed to user device 105 via link 194. In some embodiments, to enable communication between analyte sensor 108 and user device 105, the serial number of the analyte sensor must be entered into user app 160.

User app 160 may provide the ability to calculate a suggested bolus dose through the use of a bolus calculator. The bolus calculator is provided as a convenience to the user to aid in determining the suggested bolus dose based on ingested carbohydrates, most-recent blood glucose readings (or a blood glucose reading if using fingerstick), programmable correction factor, insulin to carbohydrate ratio, target glucose value, and insulin on board (IOB). IOB is estimated by user app 160 taking into account any manual bolus and insulin delivered by the algorithm.

*Description of Embodiments*

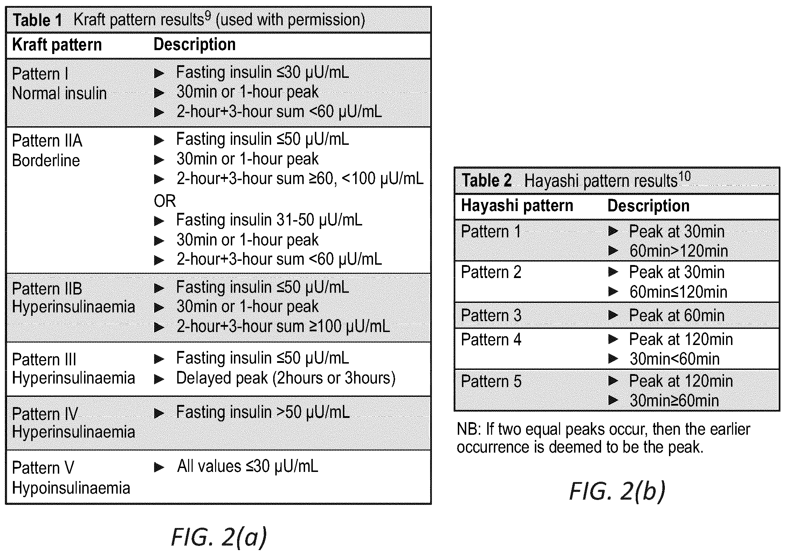

In a first embodiment of the invention, the results of a Kraft test can be used to initiate parameters of ADD system 100 for new users of the system. In the proposed embodiment, a Kraft test combines a glucose tolerance test with an insulin assay and translates the body's insulin response into an indicator of the user's insulin resistance. Specifically, a prospective new type 2 diabetes user of ADD system 100 is administered the Kraft test by a healthcare professional. The user is recommended to execute an overnight fast of at least 10 hours. The user then receives a 75g oral glucose challenge, with glucose and insulin sampling executed at initiation, and at 30min, 60min, 120min, and 180min after ingestion of the glucose challenge. The patterns of these measurements can be translated into potential insulin resistance or hyperinsulinemia by using the tables shown in **FIGS. 2(a-b)** to categorize the user into one of the patterns identified in the tables. **FIG. 2(a)** shows a table for the traditional Kraft test, while **FIG. 2(b)** shows the Hayashi patterns. It should be noted that the test administered to the user is the same, but the results of the test may be interpreted in two ways using the Kraft patterns or the Hayashi patterns.

Each pattern is predictive of a risk of increasing insulin resistance and/or hyperinsulinemia, and thus indicates that the user may require a more aggressive insulin delivery algorithm settings as administered by ADD system 100 as an initial state for users with Type 2 Diabetes. Therefore, the following different user-facing parameters of MDA 129 may be recommended for initiation of ADD system 100, depending on the outcome of either Kraft or the Hayashi pattern tests: (1) recommend a glucose target; (2) initial reduction of the total daily insulin (TDI) input parameter from manual daily injection therapy; (3) algorithm aggressiveness (e.g., referred to herein as a Q:R Ratio); and (4) basal/bolus split. In one embodiment, the adjustments made depending on the test results are shown in Table 1:

Table 1

| Kraft/Hayashi Pattern | Recommended Glucose Target (mg/dL) | Initial Reduction in TDI From MDI Therapy | Algorithm Aggressiveness (Q:R Ratio) | Basal/Bolus Split (%) |
|---|---|---|---|---|
| 1 or 5 (Kraft Only) | 110-150 | -20% | No Change | 50/50 |
| 2A (Kraft Only) | 110-150 | -20% | No Change | 50/50 |
| 2B / 2 | 110-140 | -15% | +10% | 52.5/47.5 |
| 3 | 110-130 | -15% | +15% | 55/45 |
| 4 | 110-130 | -10% | +20% | 57.5/42.5 |
| 5 (Hayashi Only) | 110-130 | -10% | +25% | 60/40 |

Note that the Q:R ratio, in ADD system 100 described herein, is an exemplary ratio of a weighting factor for a glucose cost component (Q) and a weighting factor for an insulin cost component (R) in a cost function. MDA 129 seeks to minimize the total cost (J), which is a function of these two costs of excursion (glucose level from setpoint and insulin delivery amount from a baseline insulin delivery amount). Adjustments to the coefficients Q and R can adjust the relative impact of the two cost components (glucose or insulin costs) and thus affects the aggressiveness of MDA 129 in determining the quantity of insulin to dispense. A generic cost function may be expressed as:

$$ J(I_{rec}) = Q\big(f(I_{rec}) - G_{target}\big)^n + R(I_{rec} - I_b)^m $$

where J is the total penalty, $I_{rec}$ is the current recommended insulin delivery being assessed for the total penalty, Q is the coefficient of the glucose excursions, $f(I_{rec})$ is any generic function to associate this recommended insulin delivery with a corresponding expected glucose value, $G_{target}$ is the current control target, R is the coefficient for insulin excursions, $I_b$ is the current baseline insulin delivery, and n and m are generic coefficients representing any scaling of the penalties for glucose and/or insulin excursions. For example, the cost function regards a deviation from the recommended glucose target as glucose cost and a deviation from basal insulin deliveries (e.g. the administration of a bolus) as insulin cost. The cost function may estimate one or more future glucose values of the user when given a specific dose of insulin, for example as a bolus. The cost function may then optimize the cost by determining which insulin dose results in the lowest sum of glucose cost and insulin cost. Accordingly, an increase of the glucose cost component (Q) may result in an increase in the cost of the user's predicted glucose values deviating from the recommended glucose target, in particular for an extended duration. Hence, the optimization of the cost function with a higher glucose cost component Q may result in a higher recommended dose of insulin to bring the user back to the recommended glucose target after a meal, compared to the optimization of the same cost function with a lower glucose cost component Q and vice versa. Accordingly, an increase of the insulin cost component (R) may result in an increase in the cost of insulin delivered above the basal rate, e.g. a bolus. Hence, the optimization of the cost function with a higher insulin cost component R may result in a lower recommended dose insulin to bring the user back to the recommended glucose target, compared to the optimization of the same cost function with a lower insulin cost component R and vice versa. It should be noted that the recommended glucose target may be a range of glucose values. A further explanation of the Q:R ratio and total cost (J) calculation can be found in U.S. Application No. 16/789,051, published as US 2021/0244881, which is hereby incorporated by reference in its entirety. In particular, paragraphs [0068] to [0095] of US 2021/0244881 relating to the cost function and its parameters are incorporated herein by reference.

As the increase of the glucose cost component Q relative to the insulin cost component R typically results in higher doses of insulin being administered to the user, such an increase in the Q:R ratio may be regarded as in an increase in the aggressiveness of the ADD system 100 or method(s) executed thereon. In some embodiments, the ADD system 100 or method(s) executed thereon determine an insulin bolus dose using a cost function, wherein the cost function uses the glucose cost component Q and the insulin cost component R, in particular wherein an increase of the glucose cost component Q is associated with a higher cost for the user being outside a recommended glucose target and an increase of insulin cost component R is associated with an increased cost for a higher insulin bolus dose. In some embodiments, when the Kraft pattern test identifying a 1, 2A or 5 pattern, the aggressiveness of the ADD system 100 or method is not changed and/or the ratio of Q:R is changed by less than 10%, in particular remain unchanged. Additionally or alternatively, in some embodiments, when the Kraft pattern test identifying a 1, 2A or 5 pattern, the recommended glucose target is set to between about 110 mg/dL to about 150 mg/dL, the bolus/basal split is set to between about 1.1: 1 to about 1:1.1, in particular to about 1.05: 1 to about 1: 1.05. In some embodiments, when the Kraft pattern test identifying a 2B pattern or the Hayashi pattern Test identifying a 2 pattern, the aggressiveness of the ADD system 100 or method is increased and/or the ratio of Q:R is increased by at least 5%, in particular between about 7 % to about 13 %. Additionally or alternatively, in some embodiments, based on the Kraft pattern test identifying a 2B pattern or the Hayashi pattern Test identifying a 2 pattern, the recommended glucose target is set to between about 110 mg/dL to about 140 mg/dL, the bolus/basal split is set to between about 1.05:1 to about 1.3:1, in particular to about 1.1:1 to about 1.2:1. In some embodiments, based on the Kraft pattern test identifying a 3 pattern or the Hayashi pattern Test identifying a 3 pattern, the aggressiveness of the ADD system 100 or method is increased and/or the ratio of Q:R is increased by at least 10%, in particular between about 12 % to about 18 %. Additionally or alternatively, in some embodiments, based on the Kraft pattern test identifying a 3 pattern or the Hayashi pattern Test identifying a 3 pattern, the recommended glucose target is set to between about 110 mg/dL to about 130 mg/dL, the bolus/basal split is set to between about 1.1:1 to about 1.4:1, in particular to about 1.2:1 to about 1.3:1. In some embodiments, based on the Kraft pattern test identifying a 4 pattern or the Hayashi pattern Test identifying a 4 pattern, the aggressiveness of the ADD system 100 or method is increased and/or the ratio of Q:R is increased by at least 15%, in particular between about 17 % to about 23 %. Additionally or alternatively, in some embodiments, based on the Kraft pattern test identifying a 4 pattern or the Hayashi pattern Test identifying a 4 pattern, the recommended glucose target is set to between about 110 mg/dL to about 130 mg/dL, the bolus/basal split is set to between about 1.2:1 to about 1.5:1, in particular to about 1.3:1 to about 1.4:1. In some embodiments, based on the Hayashi pattern Test identifying a 5 pattern, the aggressiveness of the ADD system 100 or method is increased and/or the ratio of Q:R is increased by at least 20%, in particular between about 22 % to about 28 %. Additionally or alternatively, in some embodiments, based on the Hayashi pattern Test identifying a 5 pattern, the recommended glucose target is set to between about 110 mg/dL to about 130 mg/dL, the bolus/basal split is set to between about 1.35:1 to about 1.7:1, in particular to about 1.45:1 to about 1.55:1. In some embodiments, a user provides the results of the Kraft test to the MDA 129 and their TDI for MDI (multiply daily injections), wherein the MDA 129 sets a new TDI for the user based on TDI for MDI and the result of the Kraft Test. In some embodiments, based on the Kraft Test identifying a 1, 2A or 5 pattern, the new TDI for the user may be between about 75% to 85%, more specifically between about 77% to about 83% of the user's TDI for MDI. In some embodiments, based on the Kraft Test identifying a 2B pattern or the Hayashi Test identifying a 2 pattern, the new TDI for the user may be between about 80% to 90%, more specifically between about 82% to about 88% of the user's TDI for MDI. In some embodiments, based on the Kraft Test identifying a 4 pattern or the Hayashi Test identifying a 5 pattern, the new TDI for the user may be between about 85% to 95%, more specifically between about 87% to about 93% of the user's TDI for MDI.

As would be realized, the adjustments shown in Table 1 can be modified in any manner, for example, on a per user basis, by personalizing the extent of the changes to various glucose control parameters listed in Table 1. For example, it may be desired to limit the maximum extent of changes in system parameters for certain users who may, due to external factors, desire more conservative insulin delivery beyond those recommended by the Kraft or Hayashi parameters. In those cases, the adjustments for the type 4 or 5 patterns may be limited to the adjustments listed for type 3. In addition, other parameters of the MDA 129 could be adjusted. Such modifications are contemplated to be within the scope of the invention.

In one embodiment of the invention, the user may provide the results of the Kraft test to MDA 129 via user app 158 of user device 105, and MDA 129 may automatically make adjustments to the parameters in Table 1 (glucose target; TDI; algorithm aggressiveness; basal/bolus split) using the quantities shown (based on the Kraft / Hayashi pattern results) or other quantities that vary up or down compared those shown in exemplary Table 1, or may be automatically make adjustments to other internal parameters.

By way of example, assume that a user has received results of a Kraft test which indicate the user has a "Pattern III" Kraft pattern or Hayashi pattern, based on **FIGS. 2(a-b).** The user may provide this result to MDA 129 via a user interface on app 158 of user device 105. The user may also provide a total daily insulin (TDI) that the user had been receiving while treating their diabetes with syringes via multiple daily injections (MDI) (typically, users require less insulin when being treated with an insulin pump that is operated by an algorithm, compared with treatment by manual injections of insulin with a syringe). For example, if the user's TDI under MDI injections was 40 Units, then the user's TDI for treatment by MDA 129 and drug delivery device 102 may be 34 Units (or 85% of 40 Units, based on Table 1 and a Kraft / Hayashi pattern of 3). Further, based on the user's Kraft / Hayashi pattern of 3, the algorithm may increase its aggressiveness by 15% (e.g., by adjusting a Q:R ratio in one type of algorithm aggressiveness calculation), and the algorithm may further adjust the basal/bolus split to be 55/45 instead of 50/50 (meaning that 55% of the user's daily insulin comes in the form of basal and 45% comes in the form of bolus insulin deliveries). These parameters may be used as the initial algorithm settings for the user's diabetes treatment, each applied as an adjustment to the default settings, as listed in row 1 of Table 1 above, based on receipt of results of the user's Kraft test.

In a second embodiment of the invention, ADD system 100 instructs the user to administer a self-guided insulin resistance test similar to the Kraft test and uses the results to adjust the parameters of MDA 129 in real time. The self-guided test is a simulated Kraft/insulin assay test and is based on the time it takes for the user's glucose concentration to return to a baseline level following ingestion of a quantity of fast-acting carbohydrates. The self-guided test may be administered periodically, via a setting in user app 160 or may be administered at the explicit request of the user. These tests can be executed at any time in which the user may desire an updated estimate of the user's glucose and insulin dynamics and can be executed for both new users of ADD systems without prior history, as well as existing users of ADD systems.

To administer the test, user app 160 provides the user with instructions via user interface 158 of user device 105. The user may be shown a screen recommending that the user fast for a predetermined fasting period, for example 10 or more hours. At the completion of the fasting period, the user can be prompted to confirm the fast and may confirm that the fast occurred by pressing a button displayed in user interface 158.

Once the user confirms the fasting period, the system can display another screen via user interface 158 to recommend ingestion of a predetermined quantity of fast-acting carbohydrates, for example, 75g (e.g., a specified amount of sugar cubes, sugary drinks, white bread, etc.), and further inform the user that an accompanying meal bolus should not and will not be delivered as part of the test. The user may be provided with suggestions for foods that may be ingested which contain fast-acting carbohydrates, and the required quantities of those foods. In addition, because individual units of suggested foods may not sum exactly to the recommended quantity (e.g., 75g) of fast-acting carbohydrates, the user may be provided an input field in user interface 158 where the exact number of fast-acting carbohydrates that user ingested may be entered.

After the user ingests the fast-acting carbohydrates, the user can indicate that the ingestion was completed by pressing a button on user interface 158. This indication from the user allows the system to begin its assessment. The user may be informed, via a user interface 158, to not ingest further carbohydrates for a predetermined post-prandial period, for example, 5 hours.

Given the relative length of time required for glucose ingestion to result in glucose rise and given the relative delay in the impact of glucose ingestion resulting in glucose rise, categorization into a pattern based on the outcome of the simulated Kraft test that the users are guided through by the ADD system may not be feasible. Particularly, it is unlikely that user will be able to manually follow the full execution of Kraft test to categorize the user's glucose responses into a pattern similar to Table 1. Instead, a determination can be made of whether the insulin delivered by MDA 129 in the post-prandial period (e.g., through automated basal deliveries of insulin by the AID algorithm in response to the observed rise in measured glucose concentrations from a CGM) subsequent to the ingestion of the fast-acting carbohydrates (e.g., 5-hours) was of a sufficient quantity to address the quantity of the fast-acting carbohydrates ingested. That is, it is determined if MDA 129 provided enough insulin during the post-prandial period to reduce the user's blood glucose level back to the user's baseline blood glucose level (e.g., 110 mg/dL) within the post-prandial period (e.g., 5 hours) subsequent to the ingestion of the fast-acting carbohydrates. In some embodiments, the length of the post-prandial period is between about 4 hours to about 12 hours.

Specifically, an Insulin Response parameter ($I_R$) can be calculated in accordance with Eq. (1) for each cycle $k$ in the post-prandial period:

$$I_R(k) = \frac{\sum_{i=1}^{60} I(k+i)}{FAC * IC}$$

(Eq. 1)

where:

*FAC* is a number of grams of fast-acting carbohydrates ingested by the user as part of the self-guided test; and

*IC* is the user's insulin to carbohydrate ratio, typically given by $\frac{600}{TDI}$ where *TDI* is the user's total daily insulin. Accordingly, in some embodiments, the Insulin Response parameter ($I_r$) is determined by determining the sum of all insulin deliveries within a post-prandial window and dividing the sum by the number of grams of fast-acting carbohydrates ingested by the user as part of the self-guided test and user's insulin to carbohydrate ratio.

It should be noted that Eq. (1) assumes that MDA 129 is operating on 5-minute cycles. That is, MDA 129 receives a new glucose reading from a continuous glucose monitor 108 every 5 minutes and recalculates all parameters regarding the insulin delivery every 5 minutes. Thus, a 5-hour period is 60 cycles, given by the upper limit of the sum in the numerator of the fraction in Eq. (1). For medication delivery algorithms using cycles other than 5-minute cycles, a different limit for the sum can be used. For example, a 3-minute cycle may result in 100 cycles in a post-prandial window of 5 hours, whereas a 10-minute cycle may result in 30 cycles during a post-prandial window of 5 hours. In some embodiments, the cycle may represent a period of time between about 1 min to about 30 min, more specifically between about 2 min to about 15 min, and in particular between about 3 min to 10 min.

A final value for the insulin response parameter is given by Eq. (2):

$$I_{R,Final} = 0.95 I_R(j-1) + 0.05 I_R(j)$$

(Eq. 2)

where:

$I_R(1) = 1$; and

*j* is the $j^{th}$ iteration of the calculation of the insulin response factor (i.e., each time the self-guided test is run). Accordingly, in some embodiments, the final value for the insulin response parameter is determined summing the values of a multiplication of a previous insulin response parameter with a first proportion factor and a multiplication of a current insulin response parameter with a second proportion factor. In some embodiments, the first proportion factor is determined as the result of subtracting the second proportion factor from 1. In some embodiments, the second proportion factor is between about 2 % to about 20 %, more specifically between about 3 % to about 10%.

The response factor can then be implemented into the TDI input parameter of MDA 129, in accordance with Eq. (3), with the caveat of potential inaccuracies in the user's carbohydrate ingestion and user non-compliance limiting the maximum adjustment at any time.

$$TDI_{Input} = I_{R,Final}\, TDI$$

(Eq. 3)

Accordingly, in some embodiments, the TDI input parameter is determined by multiplying the final value for the insulin response parameter with the TDI.

In one embodiment of the invention, the maximum adjustment may be limited to, for example, no more than 5%. In some embodiments, the maximum adjustment is between about 2 % to about 20 %, more specifically between about 3 % to about 10%. This implementation means that the TDI input parameter can be increased if the system appears to require delivery of a larger than the typical amount required to compensate for the quantity of fast acting carbohydrates ingested by the user, and *vice versa*, during the post-prandial period after the user's ingestion of indicated quantity of fast-acting carbohydrates.

In variations of this embodiment, the insulin resistance parameter ($I_{R,Final}$) could also be used to make adjustments to other parameters of MDA 129; for example, those parameters that are adjusted based on the results of the Kraft test shown in Table 1. In other variations of this embodiment, different time periods could be used. For example, the time during which the user must fast or the post-prandial period during which insulin delivery is monitored may be altered without digressing from the intended scope of the invention.

The result of the self-guided test may also be provided as a monthly output to the user or the user's healthcare professional via a health visualization application as an ongoing measurement of the user's insulin and glucose dynamics, helping the user assess any changes in their state of diabetes. Accordingly, in some embodiments, the user or healthcare professional is informed of the self-guided test result (e.g. self-guided Kraft Test or self-guided Hayashi test), more specifically after a duration of 2 weeks to about 3 month, more specifically 3 weeks to 2 month after a previous self-guided test.

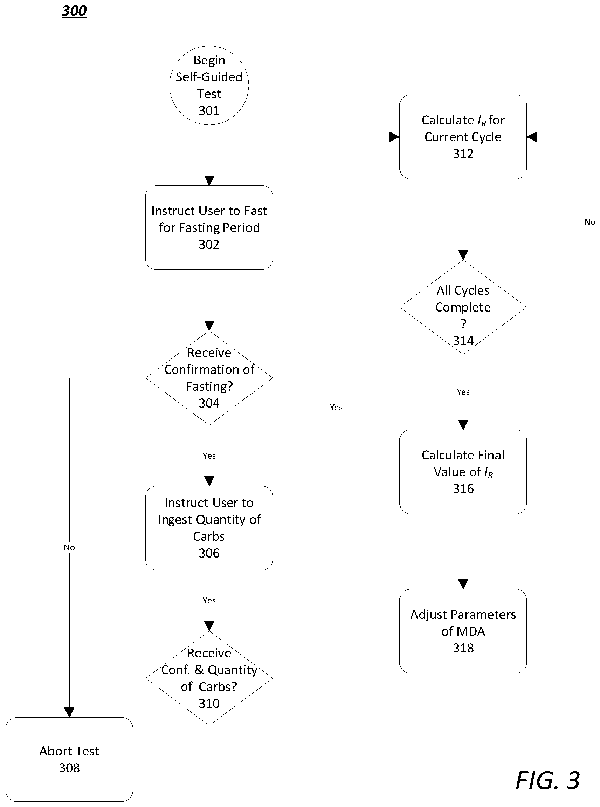

FIG. 3 is a flowchart showing the process 300 for conducting the self-guided test. The self-guided test is initiated at 301. As previously discussed, the self-guided test may be initiated periodically by MDA 129 or may be administered at the explicit request of the user. At 302, the user is instructed to fast for a predetermined period of time. In certain embodiments, the period of fasting may be 10 or more hours. In some embodiments, the period of fasting may be between about 8 hours to about 14 hours. The user may receive the instructions, for example, via a modal window displayed in the user interface 158 of user app 160. After the fasting period has expired, at decision point 304, the user may be presented with a screen, for example, a modal window in user interface 158, asking for confirmation that the user has fasted for the required fasting period. If the user fails to answer, or answers in the negative, the test is aborted at 308. However, if the user confirms the fasting was completed, the user is then instructed to ingest a pre-determined quantity of fast-acting carbohydrates at 306. Alternatively or additionally, in some embodiments, the MDA 129 applies a meal identification algorithm to identify whether the user has eaten (or consumed carbohydrates in general, e.g. sweetened drinks) within the period of feasting and based on the meal identification algorithm having identified a meal aborting the test and based on the meal identification algorithm not having identified a meal instructing the user to ingest a pre-determined quantity of fast-acting carbohydrates at 306. Applying the meal identification algorithm may be useful as users in particular when starting insulin therapy may be forgetful about meals or may not be aware that for example sweetened drink may compromise the test. Further, it is known that users (or medical patients in general) may not be complacent and still confirm that they have adhered to a medical plan. The user may be advised, for example, via a modal window displayed in user interface 158, of several different options of foods that could be ingested to provide the required quantity of fast-acting carbohydrates. At decision point 310, the user may be asked to confirm, for example, via modal window, the ingestion of the predetermined quantity of carbohydrates and, may be prompted for the exact quantity of fast-acting carbohydrates that were ingested, if the amount that was ingested differs from the predetermined suggested quantity. If no response is received from the user, or, if the user response in the negative, the test is aborted 308. If the user confirms ingestion of the carbohydrates at decision point 310, at step 312, the insulin response parameter is calculated for the current cycle. At decision point 314, if the insulin response parameter has not been calculated for all desired cycles, control returns to step 312 where the insulin response parameter is calculated for the next cycle. As explained above, in the exemplary MDA 129 described herein, each cycle is 5 minutes in duration and, as such, a 5-hour post-prandial period during which the insulin resistance parameters calculated consumes 60 cycles. As would be realized, other periods for calculating the insulin resistance parameter as well as cycles of other durations may be used. At 314, if all the cycles are complete, the process moves to step 316, where the final value of the insulin resistance parameter is calculated and, at step 318, the parameters of MDA 129 are adjusted. As previously stated, the total daily insulin (TDI) may most easily be modified; however, any other parameters of MDA 29 may be modified as well, including, for example, those listed in Table 1.

As would be realized by one of skill in the art, many variations on the embodiments disclosed herein are possible. In particular, various sizes, materials and configurations are contemplated to be the within the scope of the invention and the invention is not meant to be limited by the specific embodiments disclosed herein

The following examples pertain to various embodiments disclosed herein for the needle insertion/reduction mechanism for use with an automatic drug delivery system.

Example 1 is a first embodiment of the invention directed to a method for adjusting default parameters controlling quantities of insulin dispensed by an automatic drug delivery system for a new user comprising receiving results of a Kraft test administered on the new user of the automatic drug delivery system, the Kraft test identifying a pattern indicating a degree of insulin resistance and adjusting the default parameters of a medication delivery algorithm of the automatic drug delivery system based on the identified pattern to increase or decrease the quantity of insulin administered to the user by the automatic drug delivery system.

Example 2 is an extension of Example 1, or any other example disclosed herein, wherein the parameters comprise a recommended glucose target for the user, an initial reduction in total daily insulin from insulin delivered during manual daily injection therapy, aggressiveness of the medication delivery algorithm and a basal/bolus split.

Example 3 is a second embodiment of the invention directed to a method, in an automatic drug delivery system, for directing the user to administer a self-guided insulin resistance test comprising advising the user to fast for predetermined fasting time, advising the user to ingest a predetermined quantity of fast-acting carbohydrates, determining, for a predetermined post-prandial period after ingestion of the fast-acting carbohydrates, whether the insulin administered during the post-prandial period is sufficient to lower blood glucose levels of the user to a baseline value and, when the insulin administered during the post-prandial period is insufficient, adjusting parameters of a medication delivery algorithm to correct for insulin resistance of the user.

Example 4 is an extension of Example 3, or any other example disclosed herein, wherein advising the user to fast for the predetermined fasting time comprises providing an instruction to the user in a user interface of the automatic drug delivery system advising of the length of the fasting time.

Example 5 is an extension of Example 3, or any other example disclosed herein, wherein the predetermined fasting time is 10 or more hours.

Example 6 is an extension of Example 5, or any other example disclosed herein, wherein advising the user to ingest a predetermined quantity of fast-acting carbohydrates comprises providing an instruction to the user in the user interface of the automatic drug delivery system, the instruction including the desired quantity of fast-acting carbohydrates to be ingested.

Example 7 is an extension of example 6, or any other example disclosed herein, wherein the instruction further comprises providing suggestions of foods for the user to ingest comprising the desired quantity of fast-acting carbohydrates.

Example 8 is an extension of Example 6, or any other example disclosed herein, wherein the instruction further comprises a field allowing the user to enter the exact quantity of fast-acting carbohydrates that were ingested.

Example 9 is an extension of Example 3, or any other example disclosed herein, the method further comprising providing an instruction in the user interface of the automatic drug delivery system instructing the user to not eat during the post-prandial period.

Example 10 is an extension of Example 3, or any other example disclosed herein, the method further comprising determining, during the post-prandial period, an insulin response factor.

Example 11 is an extension of Example 10, or any other example disclosed herein, wherein determining the insulin response factor comprises aggregating the insulin response factor calculated during the post-prandial period of the current insulin response test with a final insulin response factor from a previous insulin response test.

Example 12 is an extension of Example 11, or any other example disclosed herein, wherein each cycle of the medication delivery algorithm is 5 minutes in duration.

Example 13 is an extension of claim 3, or any other example disclosed herein, wherein adjusting parameters of the medication delivery algorithm to correct for insulin resistance of the user comprises adjusting a total daily insulin input parameter of the medication delivery algorithm by the insulin response factor.

Example 14 is an extension of Example 3, or any other example disclosed herein, wherein adjusting parameters of the medication delivery algorithm comprises adjusting one or more of a recommended glucose target for the user, aggressiveness of the medication delivery algorithm and a basal/bolus split.

Example 15 is a third embodiment of the invention directed to a system comprising a drug delivery device, a blood glucose sensor in communication with the drug delivery device, a user device in communication with the drug delivery device and software executing on the user device or on the drug delivery device which causes the system to advise the user to fast for predetermined fasting time, advise the user to ingest a predetermined quantity of fast-acting carbohydrates, determine, for a predetermined post-prandial period after ingestion of the fast-acting carbohydrates, whether the insulin administered during the post-prandial period is sufficient to lower blood glucose levels of the user to a baseline value and, when the insulin administered during the post-prandial period is insufficient, adjust parameters of medication delivery algorithm to correct for insulin resistance of the user.

Example 16 is an extension of Example 15, or any other example disclosed herein, wherein the software further causes the system to provide an instruction to the user in the user interface of the user device advising of the length of the fasting time.

Example 17 is an extension of Example 15, or any other example disclosed herein, wherein the software further causes the system to provide instruction to the user in the user interface of the user device, the instruction including the desired quantity of fast-acting carbohydrates to be ingested.

Example 18 is an extension of Example 17, or any other example disclosed herein, wherein the software further causes the system to provide suggestions of foods for the user to ingest comprising the desired quantity of fast-acting carbohydrates.

Example 19 is extension of Example 15, or any other example disclosed herein, wherein the software further causes the system to aggregate the insulin response factor calculated during the post-prandial period of the current insulin response test with a final insulin response factor from a previous insulin response test.

Example 20 is extension of Example 18, or any other example disclosed herein, wherein the software further causes the system to adjust a total daily insulin input parameter of the medication delivery algorithm by the insulin response factor.

Example 21 is an extension of example 15, or any other example disclosed herein, wherein the software further causes the system to adjust one or more of a recommended glucose target for the user, aggressiveness of the medication to delivery algorithm and a basal/bolus split.

Software related implementations of the techniques described herein may include, but are not limited to, firmware, application specific software, or any other type of computer readable instructions that may be executed by one or more processors. The computer readable instructions may be provided via non-transitory computer-readable media. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components. The methods described herein may be in particular computer implemented methods. The methods described herein may in particular be executed by drug delivery systems, in particular automatic drug delivery systems.

The present disclosure furthermore relates to computer programs comprising instructions (also referred to as computer programming instructions) to perform the aforementioned functionalities. The instructions may be executed by a processor. The instructions may also be performed by a plurality of processors for example in a distributed computer system. The computer programs of the present disclosure may be for example preinstalled on, or downloaded to the medicament delivery device, management device, fluid delivery device, e.g. their storage.

To those skilled in the art to which the invention relates, many modifications and adaptations of the invention may be realized. Implementations provided herein, including sizes, shapes, ratings, compositions and specifications of various components or arrangements of components, and descriptions of specific manufacturing processes, should be considered exemplary only and are not meant to limit the invention in any way. As one of skill in the art would realize, many variations on implementations discussed herein which fall within the scope of the invention are possible. Moreover, it is to be understood that the features of the various embodiments described herein were not mutually exclusive and can exist in various combinations and permutations, even if such combinations or permutations were not made express herein, without departing from the spirit and scope of the invention. Accordingly, the method and apparatus disclosed herein are not to be taken as limitations on the invention but as an illustration thereof. The scope of the invention is defined by the claims which follow.

Although the present invention is defined in the attached claims, it should be understood that the present invention can also (alternatively) be defined in accordance with the following embodiments:

1. A method for adjusting default parameters controlling quantities of insulin dispensed by an automatic drug delivery system for a new user comprising:

   receiving results of a Kraft test administered on the new user of the automatic drug delivery system, the Kraft test identifying a pattern indicating a degree of insulin resistance; and
   adjusting the default parameters of a medication delivery algorithm of the automatic drug delivery system based on the identified pattern to increase or decrease the quantity of insulin administered to the user by the automatic drug delivery system.

2. The method of embodiment 1 wherein the parameters comprise a recommended glucose target for the user, an initial reduction in total daily insulin from insulin delivered during manual daily injection therapy, aggressiveness of the medication delivery algorithm and a basal/bolus split.

3. A method, in an automatic drug delivery system, for directing a user to administer a self-guided insulin resistance test comprising:

   advising the user to fast for a predetermined fasting time;
   advising the user to ingest a predetermined quantity of fast-acting carbohydrates;
   determining, for a predetermined post-prandial period after ingestion of the fast-acting carbohydrates whether the insulin administered during the post-prandial period is sufficient to lower blood glucose levels of the user to a baseline value; and
   when the insulin administered during the post-prandial period is insufficient, adjusting parameters of a medication delivery algorithm to correct for insulin resistance of the user.

4. The method of embodiment 3 wherein advising the user to fast for the predetermined fasting time comprises providing an instruction to the user in a user interface of the automatic drug delivery system advising of the length of the fasting time.

5. The method of embodiment 3 wherein the predetermined fasting time is 10 hours.

6. The method of embodiment 3 wherein advising the user to ingest a predetermined quantity of fast-acting carbohydrates comprises providing an instruction to the user in the user interface of the automatic drug delivery system, the instruction including the desired quantity of fast-acting carbohydrates to be ingested.

7. The method of embodiment 6 wherein the instruction further comprises providing suggestions of foods for the user to ingest comprising the desired quantity of fast-acting carbohydrates.

8. The method of embodiment 6 wherein the instruction further comprises a field allowing the user to enter the exact quantity of fast-acting carbohydrates that were ingested.

9. The method of embodiment 3 further comprising:
providing an instruction in a user interface of the automatic drug delivery system instructing the user not to eat during the post-prandial period.

10. The method of embodiment 3 further comprising:
determining, during the post-prandial period, an insulin response factor.

11. The method of embodiment 10 wherein determining a final insulin response factor comprises:
aggregating the insulin response factor calculated during the post-prandial period of the current insulin response test with a final insulin response factor from a previous insulin response test.

12. The method of embodiment 11 wherein each cycle of the medication delivery algorithm is 5 minutes in duration.

13. The method of embodiment 3 wherein adjusting parameters of the medication delivery algorithm to correct for insulin resistance of the user comprises:
adjusting a total daily insulin input parameter of the medication delivery algorithm by the final insulin response factor.

14. The method of embodiment 3 wherein adjusting parameters of the medication delivery algorithm comprises:
adjusting one or more of a recommended glucose target for the user, aggressiveness of the medication delivery algorithm and a basal/bolus split.

15. A system comprising:

a drug delivery device;
a blood glucose sensor in communication with the drug delivery device;
a user device in communication with the drug delivery device; and
software executing on the user device or on the drug delivery device which causes the system to:

advise the user to fast for a predetermined fasting time;
advise the user to ingest a predetermined quantity of fast-acting carbohydrates;
determine, for a predetermined post-prandial period after ingestion of the fast-acting carbohydrates whether the insulin administered during the post-prandial period is sufficient to lower blood glucose levels of the user to a baseline value; and
when the insulin administered during the post-prandial period is insufficient, adjust parameters of a medication delivery algorithm to correct for insulin resistance of the user.

16. The system of embodiment 15, the software further causing the system to:
provide an instruction to the user in a user interface of the user device advising of the length of the fasting time.

17. The system of embodiment 15, the software further causing the system to:
provide instruction to the user in the user interface of the user device, the instruction including the desired quantity of fast-acting carbohydrates to be ingested.

18. The system of embodiment 17, the software further causing the system to:
provide suggestions of foods for the user to ingest comprising the desired quantity of fast-acting carbohydrates.

19. The system of embodiment 15, the software further causing the system to:
aggregate the insulin response factor calculated during the post-prandial period of the current insulin response test with a final insulin response factor from a previous insulin response test.

20. The system of embodiment 15, the software further causing the system to:
adjust a total daily insulin input parameter of the medication delivery algorithm by the insulin response factor.

21. The system of embodiment 15, the software further causing the system to:
adjust one or more of a recommended glucose target for the user, aggressiveness of the medication delivery algorithm and a basal/bolus split.

**Claims**

1. A method, in an automatic drug delivery system, for directing a user to administer a self-guided insulin resistance test comprising:

   advising the user to fast for a predetermined fasting time;
   advising the user to ingest a predetermined quantity of fast-acting carbohydrates;
   determining, for a predetermined post-prandial period after ingestion of the fast-acting carbohydrates whether the insulin administered during the post-prandial period is sufficient to lower blood glucose levels of the user to a baseline value; and
   when the insulin administered during the post-prandial period is insufficient, adjusting parameters of a medication delivery algorithm to correct for insulin resistance of the user.

2. The method of claim 1 wherein advising the user to fast for the predetermined fasting time comprises providing an instruction to the user in a user interface of the automatic drug delivery system advising of the length of the fasting time, in particular wherein advising the user to ingest a predetermined quantity of fast-acting carbohydrates comprises providing an instruction to the user in the user interface of the automatic drug delivery system, the instruction including the desired quantity of fast-acting carbohydrates to be ingested.

3. The method of claim 1 or 2 wherein the instruction further comprises a field allowing the user to enter the exact quantity of fast-acting carbohydrates that were ingested.

4. The method of any one of claims 1 to 3 further comprising:
providing an instruction in a user interface of the automatic drug delivery system instructing the user not to eat during the post-prandial period.

5. The method of any one of claims 1 to 4 further comprising:

   determining, during the post-prandial period, an insulin response factor, in particular wherein
   determining a final insulin response factor comprises:
   aggregating the insulin response factor calculated during the post-prandial period of the current insulin response test with a final insulin response factor from a previous insulin response test.

6. The method of claim 5 wherein adjusting parameters of the medication delivery algorithm to correct for insulin resistance of the user comprises:
adjusting a total daily insulin input parameter of the medication delivery algorithm by the final insulin response factor.

7. The method of any one of claims 1 to 6 wherein adjusting parameters of the medication delivery algorithm comprises:
adjusting one or more of a recommended glucose target for the user, aggressiveness of the medication delivery algorithm and a basal/bolus split.

8. A system comprising:

   a drug delivery device;
   a blood glucose sensor in communication with the drug delivery device;
   a user device in communication with the drug delivery device; and

software executing on the user device or on the drug delivery device which causes the system to:

advise the user to fast for a predetermined fasting time;
advise the user to ingest a predetermined quantity of fast-acting carbohydrates;
determine, for a predetermined post-prandial period after ingestion of the fast-acting carbohydrates whether the insulin administered during the post-prandial period is sufficient to lower blood glucose levels of the user to a baseline value; and
when the insulin administered during the post-prandial period is insufficient, adjust parameters of a medication delivery algorithm to correct for insulin resistance of the user.

9. The system of claim 8, the software further causing the system to:
provide an instruction to the user in a user interface of the user device advising of the length of the fasting time.

10. The system of claim 8 or 9, the software further causing the system to:
provide instruction to the user in the user interface of the user device, the instruction including the desired quantity of fast-acting carbohydrates to be ingested.

11. The system of any one of claims 8 to 10, the software further causing the system to:
aggregate the insulin response factor calculated during the post-prandial period of the current insulin response test with a final insulin response factor from a previous insulin response test.

12. The system of any one of claims 8 to 11, the software further causing the system to:
adjust a total daily insulin input parameter of the medication delivery algorithm by the insulin response factor.

13. The system of any one of claims 8 to 12, the software further causing the system to:
adjust one or more of a recommended glucose target for the user, aggressiveness of the medication delivery algorithm and a basal/bolus split.

14. A method for adjusting default parameters controlling quantities of insulin dispensed by an automatic drug delivery system for a new user comprising:

receiving results of a Kraft test administered on the new user of the automatic drug delivery system, the Kraft test identifying a pattern indicating a degree of insulin resistance; and
adjusting the default parameters of a medication delivery algorithm of the automatic drug delivery system based on the identified pattern to increase or decrease the quantity of insulin to be administered to the user by the automatic drug delivery system.

15. The method of claim 14 wherein the parameters comprise a recommended glucose target for the user, an initial reduction in total daily insulin from insulin delivered during manual daily injection therapy, aggressiveness of the medication delivery algorithm and a basal/bolus split.

**100**

Cloud Server 112

Cloud-Based Services 111

User Device 105

Communication Interface 154

Processor 151

Output Device 155

User Interface 158

Mem 153

User App 160

MDA 129

Comm. Link 115

Accessory Device 106

Comm. I/F 174

Proc 171

UI 178

Mem 173

User App 160

Drug Delivery Device 102

UI 127

Controller 121

Comm. Interface 126

Memory 123

MDA 129

Power Source 128

Sensors 184

Reservoir 124

Drive Mechanism 125

Reservoir 124-2

Drive Mechanism 125-2

Needle/ Cannula 186

Analyte Sensor 108

UI 137

Ctl 131

Mem 132

Programming Code 136

Communication Interface 135

Sensing/Measuring Device 133

Power Source 134

193
194
191
192
195
196

*FIG. 1*

| Table 1 Kraft pattern results[9] (used with permission) | |
| --- | --- |
| **Kraft pattern** | **Description** |
| Pattern I<br>Normal insulin | ▶ Fasting insulin ≤30 µU/mL<br>▶ 30min or 1-hour peak<br>▶ 2-hour+3-hour sum <60 µU/mL |
| Pattern IIA<br>Borderline | ▶ Fasting insulin ≤50 µU/mL<br>▶ 30min or 1-hour peak<br>▶ 2-hour+3-hour sum ≥60, <100 µU/mL<br>OR<br>▶ Fasting insulin 31-50 µU/mL<br>▶ 30min or 1-hour peak<br>▶ 2-hour+3-hour sum <60 µU/mL |
| Pattern IIB<br>Hyperinsulinaemia | ▶ Fasting insulin ≤50 µU/mL<br>▶ 30min or 1-hour peak<br>▶ 2-hour+3-hour sum ≥100 µU/mL |
| Pattern III<br>Hyperinsulinaemia | ▶ Fasting insulin ≤50 µU/mL<br>▶ Delayed peak (2hours or 3hours) |
| Pattern IV<br>Hyperinsulinaemia | ▶ Fasting insulin >50 µU/mL |
| Pattern V<br>Hypoinsulinaemia | ▶ All values ≤30 µU/mL |

*FIG. 2(a)*

| Table 2 Hayashi pattern results[10] | |
| --- | --- |
| **Hayashi pattern** | **Description** |
| Pattern 1 | ▶ Peak at 30min<br>▶ 60min>120min |
| Pattern 2 | ▶ Peak at 30min<br>▶ 60min≤120min |
| Pattern 3 | ▶ Peak at 60min |
| Pattern 4 | ▶ Peak at 120min<br>▶ 30min<60min |
| Pattern 5 | ▶ Peak at 120min<br>▶ 30min≥60min |

NB: If two equal peaks occur, then the earlier occurrence is deemed to be the peak.

*FIG. 2(b)*

_300_

FIG. 3

Europäisches
Patentamt

European
Patent Office

Office européen
des brevets

# EUROPEAN SEARCH REPORT

**Application Number**

EP 23 19 3193

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2011/021898 A1 (WEI CHARLES [US] ET AL) 27 January 2011 (2011-01-27) * paragraphs [0005], [0018], [0034], [0029], [0012] * | 1-15 | INV. G16H20/17 G16H40/63 |
| Y | CN 101 102 725 A (BECTON DICKINSON CO [US]) 9 January 2008 (2008-01-09) * page 9, paragraph 3 * | 1-15 | |
| A | CN 107 073 207 A (BECTON DICKINSON CO) 18 August 2017 (2017-08-18) * the whole document * | 1-15 | |
| A | US 2012/220651 A1 (CHEVION MORDECHAI [IL] ET AL) 30 August 2012 (2012-08-30) * the whole document * | 1-15 | |
| A | RU 2 553 943 C1 (G BJUDZHETNOE OBRAZOVATEL NOE UCHREZHDENIE VYSSHEGO PROFESSIONAL NOGO) 20 June 2015 (2015-06-20) * the whole document * | 1-15 | |
| A | JP 2018 502341 A (ASEKO INC) 25 January 2018 (2018-01-25) * the whole document * | 1-15 | TECHNICAL FIELDS SEARCHED (IPC) G16H |
| A | EP 3 996 100 A1 (MEDTRONIC MINIMED INC [US]) 11 May 2022 (2022-05-11) * the whole document * | 1-15 | |

The present search report has been drawn up for all claims

| Place of search | Date of completion of the search | Examiner |
|---|---|---|
| Munich | 30 January 2024 | Laub, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

               

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 19 3193

30-01-2024

| Patent document cited in search report | | | Publication date | Patent family member(s) | | | Publication date |
|---|---|---|---|---|---|---|---|
| US 2011021898 | A1 | | 27-01-2011 | DK | 3173014 | T3 | 13-09-2021 |
| | | | | EP | 2456351 | A2 | 30-05-2012 |
| | | | | EP | 3173014 | A1 | 31-05-2017 |
| | | | | EP | 3936032 | A1 | 12-01-2022 |
| | | | | EP | 4276652 | A2 | 15-11-2023 |
| | | | | ES | 2888427 | T3 | 04-01-2022 |
| | | | | US | 2011021898 | A1 | 27-01-2011 |
| | | | | US | 2014344280 | A1 | 20-11-2014 |
| | | | | US | 2021200787 | A1 | 01-07-2021 |
| | | | | WO | 2011011739 | A2 | 27-01-2011 |
| CN 101102725 | A | | 09-01-2008 | CN | 101102725 | A | 09-01-2008 |
| | | | | CN | 101124003 | A | 13-02-2008 |
| CN 107073207 | A | | 18-08-2017 | CN | 107073207 | A | 18-08-2017 |
| | | | | EP | 3174576 | A1 | 07-06-2017 |
| | | | | EP | 4101483 | A1 | 14-12-2022 |
| | | | | ES | 2921199 | T3 | 19-08-2022 |
| | | | | JP | 2017525451 | A | 07-09-2017 |
| | | | | KR | 20170039273 | A | 10-04-2017 |
| | | | | US | 2017216524 | A1 | 03-08-2017 |
| | | | | US | 2019298920 | A1 | 03-10-2019 |
| | | | | US | 2021402092 | A1 | 30-12-2021 |
| | | | | WO | 2016019192 | A1 | 04-02-2016 |
| US 2012220651 | A1 | | 30-08-2012 | AU | 2010286054 | A1 | 08-03-2012 |
| | | | | AU | 2016244189 | A1 | 27-10-2016 |
| | | | | BR | 112012003447 | A2 | 20-03-2018 |
| | | | | CA | 2771257 | A1 | 24-02-2011 |
| | | | | CN | 102573831 | A | 11-07-2012 |
| | | | | EP | 2467135 | A2 | 27-06-2012 |
| | | | | EP | 3189836 | A2 | 12-07-2017 |
| | | | | JP | 2013502407 | A | 24-01-2013 |
| | | | | MX | 349387 | B | 05-07-2017 |
| | | | | US | 2012220651 | A1 | 30-08-2012 |
| | | | | US | 2015148413 | A1 | 28-05-2015 |
| | | | | WO | 2011021203 | A2 | 24-02-2011 |
| RU 2553943 | C1 | | 20-06-2015 | NONE | | | |
| JP 2018502341 | A | | 25-01-2018 | AU | 2015339576 | A1 | 26-05-2016 |
| | | | | CA | 2927335 | A1 | 27-04-2016 |
| | | | | EP | 3050023 | A1 | 03-08-2016 |
| | | | | EP | 3933845 | A2 | 05-01-2022 |
| | | | | HK | 1222467 | A1 | 30-06-2017 |
| | | | | JP | 6989262 | B2 | 05-01-2022 |

EPO FORM P0459

For more details about this annex : see Official Journal of the European Patent Office, No. 12/82

## EP 4 336 510 A1

### ANNEX TO THE EUROPEAN SEARCH REPORT
### ON EUROPEAN PATENT APPLICATION NO.

EP 23 19 3193

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2024

| Patent document cited in search report | | Publication date | Patent family member(s) | | Publication date |
|---|---|---|---|---|---|
| | | | JP 2018502341 A | | 25-01-2018 |
| | | | US 2016117481 A1 | | 28-04-2016 |
| | | | US 2018122504 A1 | | 03-05-2018 |
| | | | US 2019019577 A1 | | 17-01-2019 |
| | | | US 2019348166 A1 | | 14-11-2019 |
| | | | US 2020402636 A1 | | 24-12-2020 |
| | | | US 2023293012 A1 | | 21-09-2023 |
| | | | US 2023329556 A1 | | 19-10-2023 |
| | | | WO 2016069475 A1 | | 06-05-2016 |
| EP 3996100 A1 | | 11-05-2022 | CN 114446493 A | | 06-05-2022 |
| | | | EP 3996100 A1 | | 11-05-2022 |
| | | | US 2022143314 A1 | | 12-05-2022 |

For more details about this annex : see Official Journal of the European Patent Office, No. 12/82

EPO FORM P0459

**REFERENCES CITED IN THE DESCRIPTION**

**Patent documents cited in the description**

- US 7303549 B
- US 7137964 B
- US 6740059 B
- US 789051
- US 20210244881 A